(19) Europäisches Patentamt
European Patent Office
Office européen des brevets

(11) Numéro de publication : **0 505 267 A1**

(12) # DEMANDE DE BREVET EUROPEEN

(21) Numéro de dépôt : **92400714.9**

(22) Date de dépôt : **18.03.92**

(51) Int. Cl.$^5$ : **C07K 3/08,** C07D 307/89, C07H 17/04

(30) Priorité : **19.03.91 FR 9103325**

(43) Date de publication de la demande : **23.09.92 Bulletin 92/39**

(84) Etats contractants désignés : **AT BE CH DE DK ES GB GR IT LI LU NL PT SE**

(71) Demandeur : **SOCIETE NATIONALE ELF AQUITAINE**
**Tour Elf, 2, Place de la Coupole, La Défense 6**
**F-92400 Courbevoie (FR)**

(72) Inventeur : **Bonnaffe, David**
**122 Avenue de la République**
**F-75011 Paris (FR)**
Inventeur : **Lubineau, André**
**2 Allée des Près, Hameau de Rouillon**
**F-91410 Dourdan (FR)**
Inventeur : **Seris, Jean-Louis**
**1180 Chemin Vignats**
**F-64110 Jurancon (FR)**
Inventeur : **Therisod, Michel**
**21, rue des Augustins**
**F-92160 Antony (FR)**

(74) Mandataire : **Boillot, Marc**
**SOCIETE NATIONALE ELF AQUITAINE (PRODUCTION), Dept. Propriété Industrielle, Tour Elf**
**F-92078 Paris la Défense Cédex 45 (FR)**

(54) **Procédé de modification chimique des protéines.**

(57) Procédé de modification chimique des protéines à l'aide d'un réactif de formule générale

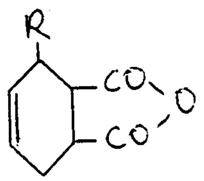

(I)

obtenu à partie du diène

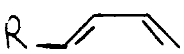

(II)

et l'anhydride maléique par addition de Diels-Alder.

EP 0 505 267 A1

Cette invention concerne un procédé de modification chimique des protéines.

La modification chimique des protéines répond à deux grandes préoccupations. Elle permet la compréhension de la structure et du fonctionnement des protéines et également le changement de ces propriétés. Ces deux volets, connaissance de la structure et surtout des sites actifs et changement de leur activité, sont particulièrement importants pour les protéines à effet catalytique que sont les enzymes.

Les techniques de modification des protéines et des parties protéiniques des enzymes sont très variées :

– Il est possible de modifier un ou plusieurs résidus d'aminoacides spécifiquement au niveau du site actif d'une enzyme, dans le but d'explorer (DS. SIGMAN Annu. Rev. Biochem 1975, 44, 899) ou modifier (E.T. KAISER Annu. Rev. Biochem. 1985, 54, 565) l'activité catalytique. On peut ranger dans cette catégorie les substrats suicides (WALSH Annu. Rev. Biochem 1984, 53, 493)

– Un seul type de résidu d'aminoacide peut être modifié par un réactif spécifique. Ce procédé décrit dans le livre "Chemical reagents for protein modification" (R.L. LUNDBLAD CRC Press, Bacaratan, USA, 1984) permet d'obtenir des informations sur le rôle du type de résidu touché.

– Utilisation de réactifs modifiant la protéine en vue de son immobilisation sur un support, par exemple greffage de groupements acryloyle puis copolymérisation (K. MARTINER Biochem. Biophys. Acta 1977, 485 1) ou la préparation des conjugués anticorps-enzymes (D.M. BOORSMA J. Immunol. Methods 1979, 30, 245).

– Préparation d'antigènes par fixation covalente d'une petite molécule sur une protéine (K. FLURKEY J. Neuroimmunol. 1985, 8 115).

– Préparation de glycoprotéines synthétiques appelées néoglycoprotéines (C.P. STOWELL Adv. Carb. Chem. Biochem 1980, 37 225).

– Changement des propriétés physico-chimiques comme la stabilité ou solubilité dans l'eau ou des solvants organiques, par réactions statistiques sur les différents sites actifs.(V. V.MOZHAEV Aur. J. Biochem. 1988, 173 147).

Nous avons trouvé maintenant un nouveau procédé de modification chimique de protéines, très versatile, permettant l'introduction de groupements extrêmement variés, aussi bien pour l'exploration des protéines que pour la modification de leurs propriétés.

Ce procédé permet l'attaque non seulement des groupes amine libres, mais également les autres fonctions à caractère nucléophile des protéines, comme les fonctions hydroxyle, thiol, thioether, imidazole ou guanidine.

Un autre avantage du procédé est la possibilité de travailler soit en milieu aqueux, soit en milieu organique, en fonction du site qu'on désire modifier.

Pour cela, le procédé de modification chimique des protéines, selon l'invention, est caractérisé en ce que la protéine est mise en contact dans un milieu liquide avec un réactif de formule générale :

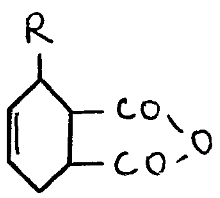

(I)

où R est un radical organique monovalent.

Le radical organique R peut être attaché au cycle directement par une liaison carbone-carbone ou par l'intermédiaire d'une liaison carbone-hétéroatome, telle qu'une liaison carbone-oxygène, carbone-azote ou carbone-soufre.

La nature du radical organique R détermine le sens de la modification. L'éventail est très large, allant de la stabilisation à l'hydrophilisation par introduction de monosaccharides et à la lipophilisation par introduction de chaînes grasses.

Le groupement anhydride du réactif (I) réagit avec les résidus nucléophiles (Nu) des protéines, comme les groupes amines, hydroxyles, thiols, imidazoles ou guanidines:

Protéines-NuH +

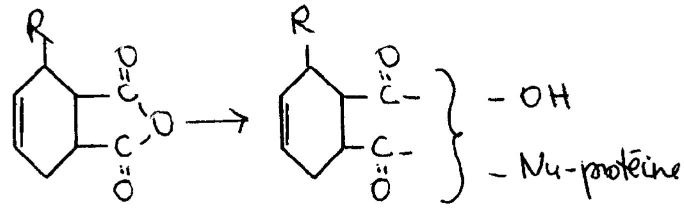

La réaction fait apparaître une nouvelle fonction carboxylique, qui peut être utilisée à son tour pour l'introduction de réactifs et renforcer ainsi l'effet recherché. Il est par exemple possible de former des amides à l'aide d'une carbodiimide soluble.

Il est évident que la modification chimique d'une protéine doit être effectuée dans des conditions assez douces pour préserver la structure et l'activité de la protéine.

Les facteurs qui interviennent principalement dans le processus de désactivation des protéines sont la température, la force ionique, le pH et la nature du solvant. La réactivité importante du composé (I) permet d'effectuer la réaction à basses températures, au besoin entre 5 et 10°C. Le pH et le solvant sont choisis de façon à favoriser la réaction considérée et à entraîner le minimum de dénaturation de la protéine. Pour chaque protéine et pour chaque réaction un compromis doit être trouvé.

Le choix du solvant peut également influencer la nature des groupes nucléophiles subissant l'attaque du réactif. En milieu aqueux l'anhydride réagit surtout avec les groupes amines libres. Certains solvants organiques favorisent en outre l'attaque des groupes hydroxyles, tandis que la réactivité des groupes amines diminue en milieu solvant organique.

On peut supposer que la conformation de la protéine n'est pas la même en solution aqueuse ou en suspension dans un solvant organique.

Dans ces solvants certains groupements amines sont peut-être inaccessibles ou ionisés, donc non réactifs.

Le choix du solvant peut être une façon supplémentaire de moduler la transformation chimique d'une protéine.

Le réactif (I) peut être commodément préparé par réaction Diels-Alder de l'anhydride maléique et d'un diène de formule générale :

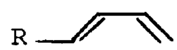 (II)

où R a la signification précitée.

La synthèse du diène, ainsi que son addition de Diels-Alder sur l'anhydride maléique se déroulent dans des conditions douces. Il devient ainsi possible d'introduire des groupements R très variés dans la protéine. L'introduction directe de ces radicaux R sur un anhydride ne serait souvent pas possible.

La réaction Diels-Alder se déroule en solvant organique.

L'anhydride maléique étant le dienophile le plus réactif, favorise la réaction et permet son déroulement dans des conditions douces. Si le diène utilisé est activé par la présence de groupes électron-donneurs, la réaction de cycloaddition se déroule même à la température ambiante.

La synthèse du diène (II) dépend bien entendu de la nature du radical organique R. Nous allons décrire à titre d'exemple, la synthèse d'un diène renfermant une molécule de glucose et celle d'un diène renfermant des esters d'acides gras. Ces deux exemples prouvent la grande versatilité du procédé, qui permet d'introduire aussi bien des groupements hydrophiles que lipophiles.

La synthèse du diène où R est une molécule de glucose est décrite par A.LUBINEAU (J. Org.Chem. 52,1987 1001). Cette synthèse comporte la réaction de l'acétobromoglucose avec le sel de sodium du malonaldehyde puis avec le méthylidène triphénylphosphorane. Le diène obtenu réagit sous forme de dérivé triméthylsilyle avec l'anhydride maléique pour donner le réactif (I) où R est une molécule de glucose persilylé.

La réaction avec une protéine permet la synthèse de néo-glucoprotéines, renfermant un nombre important de molécules de glucose donc à caractère hydrophile très renforcé.

Le réactif (I) glucosé peut être utilisé pour la modification des protéines aussi bien en milieu aqueux qu'en milieu organique. Nous l'avons utilisé à titre d'exemple pour la modification de deux protéines, l'albumine de sérum bovin (BSA) et une enzyme d'usage courant, la peroxydase du raifort (HRP) en milieu aqueux ou organique.

En milieu aqueux, la protéine est dissoute dans un tampon de pH 8 de force ionique suffisante. Le réactif insoluble dans le milieu est ajouté par petites portions. La réaction est suivie par dosage des fonctions amines libres résiduelles de la protéine. Les fonctions hydroxyles des sucres sont régénérées par hydrolyse spontanée

des groupements silyles dans l'environnement aqueux. Après réisolement de la protéine, on soumet celle-ci à un dosage des fonctions amine libres, puis après hydrolyse acide, on dose spécifiquement le glucose fixé, par une méthode enzymatique classique.

Dans le cas de la BSA, le traitement a fait disparaître la totalité des fonctions amine libre titrables, ce qui correspond à une vingtaine de groupes amine et permet la fixation de 80 molécules de glucose par molécule de protéine.

Comme la BSA ne comporte que 66 lysines, donc 66 groupes amines, d'autres résidus ont été touchés par réaction sur (I), impliquant des fonctions SH, OH, imidazole ou guanidine.

Dans le cas de la HRP, le traitement fait disparaître toutes les fonctions amine libre (5,5) et permet la fixation de 4 molécules de glucose par molécule d'enzyme. L'activité spécifique de l'enzyme est récupérée à 100%.

Il est également possible d'effectuer la réaction en milieu organique. Le choix du solvant dépend de la nature de l'enzyme à traiter.

On peut utiliser par exemple des solvants usuels des réactions d'acylation, comme la pyridine, le tetrahydrofurane ou la diméthylformamide. Il se trouve que les meilleurs solvants pour les réactions d'acylation, comme la pyridine et la diméthylformamide, sont aussi les plus dénaturants pour les protéines.

Ils peuvent être utilisés cependant pour les protéines suffisamment résistantes.

Pour la HRP, un mélange de tétrahydrofurane et de pyridine est un compromis acceptable. Dans ce cas, le réactif est soluble dans le milieu et la protéine, insoluble, reste en suspension. La réaction terminée, les groupements silyles sont hydrolysés. Après réisolement de la protéine, on dose les fonctions amine libre et les sucres fixés. Le traitement laisse subsister deux fonctions amine résiduelle sur les six existantes et permet de fixer vingt molécules de glucose par molécule d'enzyme. L'activité spécifique est récupérée à 20%.

La réaction en milieu organique a ainsi permis la fixation, de vingt molécules de glucose par molécule d'enzyme, contre 3-4 en phase aqueuse. Le nombre d'unités de glucose fixées croît linéairement avec la proportion de pyridine.

Par contre, en même temps l'activité spécifique diminue également de façon linéaire. Pour chaque enzyme un compromis doit donc être trouvé et une mise au point est nécessaire.

Le traitement par le réactif (I) fait apparaître une nouvelle fonction carboxylique par résidu de glucose fixé. On peut utiliser ces nouvelles fonctions carboxyliques pour fixer par exemple des composés aminés à l'aide d'une carbodiimide soluble. Nous avons ainsi fixé sur la BSA de la glucosylamine, selon la méthode décrite par MOCZAR et VASS (Carboydr. Res. 1976 50 133). L'hydrolyse acide de ce produit démontre la présence de 105 molécules de glucose par molécule de protéine.

Le traitement à la glucosylamine, de la BSA elle-même, ne permet de fixer que 4 à 5 glucosylamines sur les 100 fonctions acide carboxylique que comporte la protéine.

Un autre exemple de modification chimique de protéines par le procédé selon l'invention, est l'introduction de chaînes d'acides gras pour rendre la protéine lipophile.

Le réactif est préparé par réaction du diméthylcétal de l'aldéhyde glycérique avec l'allylidène triphényl phosphorane. Après hydrolyse du cetal, le diol obtenu est estérifié par le chlorure de stéaryle.

Le diène réagit avec l'anhydride maléique pour donner la réactif (I) où

$$R = \begin{array}{l} CH-O-CO(CH_2)_{16}CH_3 \\ | \\ CH_2-O-CO(CH_2)_{16}CH_3 \end{array}$$

Ce composé a été utilisé pour modifier la BSA et la superoxyde dismutase (SOD). La réaction a lieu en milieu aqueux. Après isolement de la protéine, on dose les amines libres résiduelles, puis après hydrolyse alcaline on détermine par HPLC le nombre de molécules d'acide stéarique fixées.

Sur la BSA, le traitement a pour premier effet de rendre la protéine insoluble dans l'eau, ce qui empêche le dosage des fonctions amine libres. L'hydrolyse alcaline libère 140 molécules de stéarate par molécule de protéine, correspondant à 70 molécules de réactif (I) fixées.

Sur la SOD, le même traitement fait disparaître la totalité des fonctions amine titrables. L'activité spécifique résiduelle est de 15%.

Il est bien entendu que ces deux exemples, décrits plus en détail dans la partie expérimentale ne limitent en rien la portée de l'invention.

**EXEMPLES**

– Dosage de protéines : par la méthode de l'acide bicinchoninique (P.K. Smith Anal.Biochem. 1985 150 75) et/ou par mesure de l'absorbance à 276nm.La divergence entre les deux méthodes ne dépasse pas 10%.

– Dosage des fonctions $NH_2$ libres : par la méthode à l'acide trinitrophénylsulfonique. (R. Field Biochem.J. 1972 124 581).

– Dosage du glucose fixé : 1 à 3mg de protéine modifiée purifiée sont hydrolysés par 1ml d'HCl 2M une heure à 100°C dans un tube à vis fermé après purge de l'oxygène par balayage à l'azote.

L'acide est neutralisé par passage sur une résine Dowex 1-x8 (200 mesh), sous forme formiate (2ml). Le glucose contenu dans l'éluat est dosé par la méthode à l'hexokinase/glucose-6-phosphate déshydrogénase (Méthods in Carbohydrate Chem. III 135 (1980).

**EXEMPLE A** (Synthèse du diène A; I,R=glucose silylé)

A.1. Le diène:glucose $-O-CH=CH-CH=CH_2$ a été préparé à partir d'acétobromoglucose par la méthode décrite par A.LUBINEAU (J. Org.Chem. 1987 52 1001).

La réaction de l'acétobromoglucose dans le diméthylsulfoxide avec le sel de sodium du malonaldehyde donne 56% d'un aldéhyde insaturé. L'addition de méthylènetriphenylphosphorane à cet aldéhyde à -78°C dans un mélange oxolanetoluène donne 83% du dérivé diénique du glucose en configuration bêta.

La même réaction à la température ambiante donne 72% du dérivé diénique en configuration alpha. Le diène (I) R=glucose est obtenu après désacetylation par un mélange 1:8:1 de triéthylamine, méthanol et eau avec un rendement pratiquement quantitatif.

A.2. Le diène de l'exemple A.1 est dissous dans 3ml de pyridine anhydre. On ajoute 5,1ml (24mmol) d'hexamethyldisilazane (HMDS) puis 0,3ml (3,9mmol) d'acide trifluoroacétique. Après 6 heures à température ambiante, le mélange est évaporé puis le produit distillé au tube à boule.

$R^t$=1,45g(93%); $E_{0,1mm}$=130-140°C; liquide incolore

$(\alpha)_D^{20}$ = -5,80° (C 1,2, $CH_2Cl_2$)

Analyse élémentaire:
pour $C_{22}H_{48}O_6Si_4$,
Calculé : C 50,72% H 9,29%
Trouvée : C 50,90% H,9,07%

A.3. A 1,45g (2,8mmol) du diène Silylé de l'exemple A.2 en solution dans 1,8ml de THF anhydre on ajoute 274mg (2,8mmol) d'anhydride maléique fraîchement distillé.

Après 12 heures à 40°C, la réaction est terminée et on ajuste le volume avec du THF anhydre de façon à obtenir une solution 0,8M qui sera utilisée telle quelle. Ce produit est un mélange de trois stéréo-isomères.

**EXEMPLE B** - (Synthèse du diène B;I,R = $-CH(-OCO(CH_2)_{16}-CH_3)-CH_2OCO(CH_2)_{16}-CH_3$)

B.1. - Préparation du diène $HO-CH_2-CH(OH)-CH=CH-CH=CH_2$ selon A. LUBINEAU et al., J. Chem. Soc. Perkin trans. 1 3011(1990).

Le diméthylcetal de l'aldéhyde glycérique (8.55g,66mmol) est ajouté à -78°C à une solution de l'allylidène phosphorane (66mmol) dans le THF (350ml) pour donner 72% du diène protégé sous forme d'un mélange cis-trans. Le diène cis n'étant pas réactif en réaction de Diels-Alder, il peut être quantitativement isomérisé en diène trans par réaction avec une quantité catalytique d'iode. Finalement le cetal est hydrolysé par contact avec une résine DOWEX $50H^+$ dans un mélange $EtOH-H_2O$ (9:1).

$(alpha)_D^{20}$-43° (c 1,8,EtOH)

Analyse élémentaire :
pour $C_6H_{10}O_2$,
calculé : C,63,36 : H,8,83 : O,28,03.
Trouvé : C, 63,19,H,8,30 : O,27,94.

B.2. - 470mg du diène préparé selon B.1 sont dissous dans 10ml de pyridine anhydre; on ajoute goutte à goutte 3ml de chlorure de stéaroyle (10,4mmol). Après 2 heures le mélange est dilué par 100ml de dichlorométhane. La phase organique est lavée par l'acide chlorhydrique dilué, séchée puis le solvant est évaporé.

Le produit est purifié par flash-chromatographie (100ml de silice; toluène).

Solide amorphe (paraffine blanche);

$R^t$:2,19g (78%); F=41-49°C; (alpha) $\frac{20}{D}$ + 9,3°C (c=1,73; $CH_2Cl_2$)
Analyse élémentaire :
pour $C_{42}H_{78}04$,
Calculé : C 77,96% H 12,15% O 9,80%
Trouvée : C 77,97% H 12,24% O 9,99%

B.3 - 1,61g du diène stéarylé de l'exemple B.2 et 243mg d'anhydride maléique fraîchement distillé sont dissous dans 0,5ml de THF anhydre. Après 24 heures à 70°C, on ajuste le volume par du THF anhydre de façon à obtenir une solution 0,75M du composé I stéarylé qui sera utilisée telle quelle. Mélange 1:1 de deux stéréo-isomères : Point de fusion et pouvoir rotatoire non déterminés puisque l'on est en présence d'un mélange stéréo-isomères.
Analyse élémentaire :
pour $C_{46}H_{80}O_7$,
Calculée : C 74,15% H 10,82% O 15,03%
Trouvée : C 74,38% H 10,80% O 14,82%

**EXEMPLE 1** (Modification de la BSA par A en milieu aqueux)

5mg de BSA (5 micro mol de $NH_2$) sont dissous dans 3ml de tampon phosphate pH 8, 0,07M. On ajoute 400 micro l de solution 0,8M de A dans le THF (0,32mmol) par fractions de 100 micro l à 1 heure d'intervalle. La réaction a lieu sous agitation sur un shaker rotatif. Après 18 heures à 7°C, on abaisse le pH à 3 pour accélérer l'hydrolyse des silyles (La BSA modifiée précipite). Après 12 heures, le pH est ajusté à 7 (la BSA se redissout) et la solution est extraite par 1ml de dichlorométhane. La protéine est purifiée par passage sur colonne de Sephadex G50 (50x2,1cm; éluant : tampon phosphate pH 7 0,02M ; 0,5ml/min).

Les fractions contenant la protéine sont dialysées contre de l'eau distillée puis lyophilisées. On récupère 80-90% de la protéine mise en oeuvre. On a fixé 80 molécules de A par molécule de protéine.

**EXEMPLE 2** (Modification de la HRP par A en milieu aqueux)

12mg d'HRP (0,28 micro mole de fonction $NH_2$) sont dissous dans 2ml de tampon phosphate pH 8,0,07M, 0,1% en phénol.

On ajoute 0,5ml d'acétone puis 200 micro l de solution d'anhydride en quatre fractions de 50 micro 1 à 1 heure d'intervalle. L'expérience est ensuite menée comme décrit pour la BSA. On récupère environ 60% de la protéine mise en oeuvre. On a fixé 4 molécules de A par molécule de protéine.

**EXEMPLE 3** (Modification de l'HRP par A en solvant organique)

A (50mg d'HRP lyophilisée on ajoute 1ml de solution 0,8M de A dans le THF et 1ml de mélange THF : pyridine 60:40.

La suspension est vigoureusement agitée pendant 48 heures à 37°C sur un shaker rotatif. On ajoute ensuite 18ml de tampon phosphate 0,07M, pH 7 0,1% en phénol. Après 4 heures à 7°C, on abaisse le pH à 3 par addition d'HCl 6M (pour accélérer l'hydrolyse des groupements silyles).

Après 2 heures à 7°C, la protéine est séparée des solvants organiques par passage sur Séphadex G 25 medium (21x3 cm ; élution: tampon phosphate 0,02M,pH 7). A la solution de protéine on ajoute trois équivalents d'hémine. Après 18 heures à 4°C, la solution est lyophilisée et le résidu repris par 5ml d'eau bidistillée. L'enzyme modifiée est purifiée par passage sur Séphadex G 50 comme décrit ci-dessus. On récupère environ 80% de la protéine mise en oeuvre. On a fixé 20 molécules de A par molécule d'enzyme.

**EXEMPLE 4** (Modification de la BSA par B en milieu aqueux)

4mg de BSA ($6.10^{-2}$ mmol de fonction $NH_2$) sont dissous dans 1ml de tampon phosphate 0,5M pH 8 - THF (80:20). La solution étant soumise à une agitation magnétique vigoureuse, on ajoute en quatre fractions, à une heure d'intervalle 120 micro l d'une solution 0,57M de B dans le THF. On laisse la réaction se poursuivre à 7°C pendant 18 heures. La protéine modifiée précipite. Elle est centrifugée 30 minutes à 0°C et rincée par deux fois avec 0,5ml d'un mélange THF : eau 20:80. On a fixé 70 molécules de B par molécule de protéine.

**EXEMPLE 5** (Couplage de la glucosylamine sur la BSA modifiée par A)

6,6mg de BSA modifiée par A sont dissous dans 0,15ml d'eau. On ajoute 22mg de glucosylamine synthétisé selon M.C.A. LOBRY de BRUYN (Rec. Trav. Chim. Pays-Bas 1985 12 98) et on ajuste le pH à 4-5 par addition d'acide phosphorique.

En cinq fois, pendant 1 heure, on ajoute 30mg d'EDC puis encore 22mg de glucosylamine tout en maintenant le pH à 4-5. On laisse le mélange agité à 7°C pendant 15 heures, puis on arrête la réaction par ajout de 30ml d'acide acétique. La protéine est purifiée par chromatographie sur Séphadex PD 10, élution tampon acétate 0,05M pH 4,6. On a fixé 25 molécules de glucosylamine sur la BSA modifié, ce qui correspond à 105 molécules de glucose par molécule de protéine.

## Revendications

**1 -** Procédé de modification chimique des protéines caractérisé en ce que la protéine est mise en contact avec un réactif de formule générale :

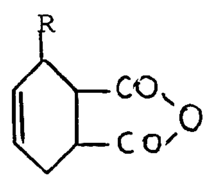

(I)

où R est un radical organique monovalent.

**2 -** Procédé selon la revendication 1 caractérisé en ce que la protéine est mise en contact avec le réactif (I) en milieu aqueux.

**3 -** Procédé selon la revendication 1 caractérisé en ce que la protéine est mise en contact avec le réactif (I) dans un solvant organique.

**4 -** Procédé selon la revendication 3 caractérisé en ce que le solvant organique est la pyridine, le tétrahydrofurane, le diméthylformamide ou leurs mélanges.

**5 -** Procédé selon l'une des revendications 1 à 4 caractérisé en ce que la température est comprise entre 5 et 10°C.

**6 -** Procédé selon l'une des revendications 1 à 5 caractérisé en ce que le réactif de formule générale (I) est préparé par addition Diels-Alder d'un diène de formule générale

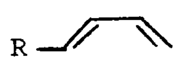

(II)

où R a la signification précitée sur l'anhydride maléique.

**7 -** Procédé selon l'une des revendications 1 à 6 caractérisé en ce que R est une molécule du glucose.

**8 -** Procédé selon l'une des revendications 1 à 6 caractérisé en ce que R est un radical distéarylglycol.

Office européen des brevets

# RAPPORT DE RECHERCHE EUROPEENNE

Numero de la demande

EP 92 40 0714

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | JOURNAL OF ORGANIC CHEMISTRY, vol. 52, 1987, Washington, D.C., US, pages 1001-1007; LUBINEAU et al.: "Aqueous cycloadditions using glyco-organic substrates. 1. Stereochemical course of the reaction"<br>* Pages 1001-1002 (Introduction) *<br>--- | 1-8 | C 07 K 3/08<br>C 07 D 307/89<br>C 07 H 17/04 |
| A | US-A-3 198 810 (WYGANT et al.)<br>* Colonne 1 *<br>----- | 1-8 | |
| | | | **DOMAINES TECHNIQUES RECHERCHES (Int. Cl.5)** |
| | | | C 07 K<br>C 07 D<br>C 07 H<br>C 07 C |

Le présent rapport a été établi pour toutes les revendications

| Lieu de la recherche | Date d'achèvement de la recherche | Examinateur |
|---|---|---|
| LA HAYE | 19-06-1992 | KORSNER S.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)